# EUROPÄISCHE PATENTSCHRIFT

(11) **EP 2 205 660 B1**
(45) Veröffentlichungstag und Bekanntmachung des Hinweises auf die Patenterteilung: **13.06.2012**
(21) Anmeldenummer: 08835337.0
(22) Anmeldetag: 01.10.2008
(51) Int. Cl.: C08G 73/04, D21H 17/56

(54) **ETHYLENIMINEINHEITEN ENTHALTENDE AMINOALKYLVINYLETHER, VERFAHREN ZU IHRER HERSTELLUNG UND IHRE VERWENDUNG**
AMINO ALKYL VINYL ETHERS CONTAINING ETHYLENE IMINE UNITS, METHOD FOR THE PRODUCTION THEREOF, AND THE USE THEREOF
AMINOALKYL VINYLETHERS CONTENANT DES UNITES ETHYLENEIMINE, LEUR PROCEDE DE PREPARATION ET LEUR UTILISATION

(30) Priorität: 04.10.2007 EP 07117909
(43) Veröffentlichungstag der Anmeldung: 14.07.2010
(73) Patentinhaber: BASF SE, 67056 Ludwigshafen (DE)
(72) Erfinder: FEUERHAKE, Robert, 68163 Mannheim (DE); SCHÖNFELDER, Daniel, 68161 Mannheim (DE); HÄHNLE, Hans-Joachim, 67435 Neustadt (DE); BRUCHMANN, Bernd, 67251 Freinsheim (DE); URIBE AROCHA, Paola, 68163 Mannheim (DE); KRAUS, Alexander, 83552 Evenhausen (DE); HÜBSCH, Christian, 83703 Gmund (DE); STAAL, Bastiaan Bram Pieter, 68161 Mannheim (DE); BAUMGÄRTNER, Ralph, 68165 Mannheim (DE)
(74) Vertreter: Peatfield, Jeremy William
(86) Internationale Anmeldenummer: PCT/EP2008/063111
(87) Internationale Veröffentlichungsnummer: WO 2009/043860

(56) Entgegenhaltungen:
- WO-A-01/36500
- US-A- 2 727 019
- DATABASE CAPLUS [Online] CHEMICAL ABSTRACTS SERVICE, COLUMBUS, OHIO, US; BEKTENOV, N. A. ET AL: "New anion exchangers from a copolymer of glycidyl methacrylate and vinyl ether of monoethanolamine" XP002516263 gefunden im STN Database accession no. 2001:548022 & IZVESTIYA MINISTERSTVA OBRAZOVANIYA I NAUKI RESPUBLIKI KAZAKHSTAN, NATSIONAL'NOI AKADEMII NAUK RESPUBLIKI KAZAKHSTAN, SERIYA KHIMICHESKAYA , (6), 3-8 CODEN: IMSKFR; ISSN: 1025-9341, 2000,

## Beschreibung

Die Erfindung betrifft Ethylenimineinheiten enthaltende Aminoalkylvinylether der Formel

H₂C = CH - O - X - NH - [Al-]ₙ - H (I),

worin
- [Al-]ₙ: für n Ethylenimineinheiten steht,
- n: eine Zahl von 2 bis 20 bedeutet und
- X: für eine C₃- bis C₄-Alkylengruppe steht,
sowie Salze und Quatemierungsprodukte der Monomeren I, Verfahren zu ihrer Herstellung und ihre Verwendung als Monomere zur Herstellung von Polymeren für den Einsatz in der Papierindustrie, als antimikrobielle Beschichtungsmittel, in Detergentien und zur Behandlung von Metalloberflächen.

US 2,727,019 offenbart Vinyloxyalkylaminoalkylamine der Formel CH₂=CHOZNHANH₂, worin A eine Alkylengruppe von 2 bis 3 C-Atomen mit mindestens 2 C-Atomen zwischen den Stickstoffatomen ist, und Z eine Alkylenkette mit mindestens 2 C-Atomen oder eine Alkylenkette von 2 C-Atomen mit einem Phenyl-, alicyclischen Hydrocarbon, Vinyl- oder Alkylsubstituenten ist, wobei dieser Alkylensubstituent Teil der Alkylenkette mit nicht mehr als 18 C-Atomen ist.

Aus der WO-A-01/36500 sind Alkylenimineinheiten enthaltende Monomere der Formel 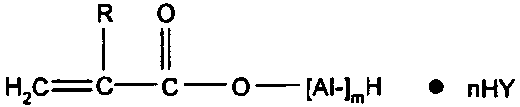 bekannt, worin
- R: für Wasserstoff oder C₁- bis C₄-Alkyl steht,
- [Al-]ₘ: eine lineare oder verzweigte Oligoalkyleniminkette mit m Alkylenimineinheiten bedeutet,
- m: für eine ganze Zahl im Bereich von 1 bis 20 steht, und das Zahlenmittel m in den Oligoalkyleniminketten wenigstens 1,5 beträgt,
- Y: das Anionäquivalent einer Mineralsäure bedeutet und
- n: für eine Zahl von 1 ≤ n ≤ m steht.

Monomere bzw. Monomergemische, bei denen in der oben angegebenen Formel das Zahlenmittel von m wenigstens 2,1, meistens 2,1 bis 8 beträgt, sind bevorzugt. Sie sind dadurch erhältlich, dass man eine ethylenisch ungesättigte Carbonsäure mit einem Oligoalkylenimin, vorzugsweise in Form eines Oligomerengemisches, umsetzt. Das dabei anfallende Produkt kann gegebenenfalls mit einer Mineralsäure HY in das Säureadditionssalz überführt werden. Solche Monomere können in einem wässrigen Medium in Gegenwart eines Initiators, der eine radikalische Polymerisation auslöst, zu Homo- und Copolymerisaten polymerisiert werden, die als Hilfsmittel bei der Papierherstellung verwendet werden.

Der Erfindung liegt die Aufgabe zugrunde, weitere Alkylenimineinheiten aufweisende Monomere zur Verfügung zu stellen.

Die Aufgabe wird erfingdungsgemäß gelöst mit Alkylenimineinheiten enthaltenden Aminoalkylvinylethem der Formel

H₂C = CH - O - X - NH - [Al-]ₙ - H (I),

worin
- [Al-]-ₙ: für n Ethylenimineinheiten steht,
- n: eine Zahl von 2 bis 20 bedeutet und
- X: für eine C₃- bis C₄-Alkylengruppe steht,
sowie Salze der Monomeren I mit Mineralsäuren oder organischen Säuren und Quatemierungsprodukte der Monomeren I mit Alkylhalogeniden oder Dialkylsulfaten.

Bevorzugt sind Alkylenimineinheiten enthaltende Aminoalkylvinylether der Formel I, worin
- [Al-]ₙ: für n Ethylenimineinheiten steht,
- n: eine Zahl von 3 bis 15 bedeutet und
- X: für die Gruppe - CH₂ - CH₂ - CH₂ - steht,

Im Fall der Salze der Monomeren I mit Mineralsäuren oder mit organischen Säuren liegt wenigstens eines der darin enthaltenen Stickstoffatome, vorzugsweise mehrere oder alle (d.h. n+1 in Formel I) der darin enthaltenen Stickstoffatome in protonierter Form vor. Sie können daher partiell oder vollständig neutralisiert sein. Zur Salzbildung kommen beispielsweise Mineralsäuren wie Salzsäure, Schwefelsäure, Phosphorsäure, Phosphonsäure oder Salpetersäure oder organische Säuren wie Ameisensäure, Essigsäure, Propionsäure, Benzolsulfonsäure, Amidosulfonsäure oder p-Toluol-sulfonsäure in Betracht.

Die Quaternierungsprodukte der Monomeren I sind durch Umsetzung der Monomeren I mit Quatemierungsmitteln wie C₁- bis C₁₈-Alkylhalogeniden oder Dialkylsulfaten zugänglich. Mindestens ein Stickstoffatom der in den Monomeren I enthaltenen Stickstoffatome liegt in den Quaternierungsprodukten in quaternierter Form vor. Quaternierte Monomere I enthalten beispielsweise 2 bis maximal n+1 quaternierte Stickstoffatome, wobei n die oben in Formel I angegebene Bedeutung hat. Als Quatemierungsmittel eignen sich beispielsweise Methylchlorid, Ethylchlorid, n-Propylchlorid, Isopropylchlorid, n-Butylchlorid, sec.-Butylchlorid, n-Hexylchlorid, Cyclohexylchlorid, Benzylchlorid, Methylbromid, Methyljodid, Ethylbromid, Ethyljodid, Dimethylsulfat und Diethylsulfat.

Alkylenimineinheiten enthaltende Aminoalkylvinylether der Formel I sind dadurch erhältlich, dass man an Amino-C₂- bis C₆-alkylvinylether mindestens ein Alkylenimin addiert, wobei man pro Mol des Vinylethers mindestens 1 Mol eines Alkylenimins einsetzt, und die Additionsprodukte zur Herstellung der Salze mit einer Mineralsäure oder einer organischen Säure neutralisiert und sie zur Herstellung der Quaternierungsprodukte mit C₁- bis C₁₈-Alkylhalogeniden oder Dialkylsulfaten umsetzt.

In einer bevorzugten Ausführungsform des erfindungsgemäßen Verfahrens addiert man an Amino-C₃- bis C₄-alkylvinylether Ethylenimin, wobei man pro Mol des Vinylethers 2 bis 20 Mol Ethylenimin anlagert.

Besonders bevorzugt sind die Reaktionsprodukte von Aminopropylvinylether mit Ethylenimin im Molverhältnis 1 : 3 bis 15. Sie zeigen im ¹H NMR-Spektrum ein Multiplet bei 2,54 bis 2,78 ppm, das die Signale der Protonen, die zur Aminogruppe im Aminoalkylvinylether benachbart sind (-CH₂-NH₂) und die Signale der Protonen dieser Gruppe in der mit Ethylenimin oligomerisierten Form. Der mit Ethylenimin oligomerisierte Aminopropylvinylether hat beispielsweise bei einem Oligomerisierungsgrad von n = 6,8 eine kationische Ladungsdichte von 9,0 meq/g (gemessen in wässriger Lösung bei einem pH-Wert von 8, einer Konzentration von 0,1 g/kg und einer Temperatur von 20 °C.

Die Umsetzung von Alkylvinylethern mit Alkyleniminen wird vorzugsweise in wässrigem Medium in Gegenwart eines Katalysators, beispielsweise einer Mineralsäure, durchgeführt. Vorzugsweise verwendet man Schwefelsäure als Katalysator. Man lässt beispielsweise ein Mol eines Katalysators wie Schwefelsäure zunächst mit 60 bis 85 Mol, vorzugsweise 70 bis 75 Mol eines Aminoalkylvinylethers reagieren und lagert dann Ethylenimin an den Aminoalkylvinylether an, der partiell als Ammoniumsalz vorliegt. Der pH-Wert des Reaktionsgemisches liegt beispielsweise in dem Bereich von ≥ 6,5, vorzugsweise bei 8 bis 11. Die Reaktionstemperatur beträgt beispielsweise 55 bis 105 °C. Sie liegt meistens in dem Temperaturbereich von 60 bis 80 °C, insbesondere 65 bis 75 °C.

Die Addition von Alkyleniminen, vorzugsweise von Ethylenimin an Aminoalkylvinylether wird beispielsweise so durchgeführt, dass man bei Raumtemperatur mindestens einen Aminoalkylvinylether in Wasser löst, zu der wässrigen Lösung einen Katalysator wie Schwefelsäure zugibt, die Mischung dann unter Rühren auf die Reaktionstemperatur erhitzt und das Alkylenimin kontinuierlich oder portionsweise unter ständigem Rühren so in die Vorlage dosiert, dass das Alkylenimin kontrolliert abreagieren und die Reaktionstemperatur geregelt werden kann. Die dafür erforderliche Reaktionszeit beträgt im Labormaßstab in Abhängigkeit von der Größe des Ansatzes beispielsweise 10 Minuten bis 3 Stunden. Der Endpunkt der Reaktion, d.h. der Zeitpunkt, an dem im Reaktionsgemisch kein Alkylenimin mehr nachgewiesen werden kann, wird mit Hilfe der Reaktion von Ethylenimin mit 4-(4-Nitrobenzyl)pyridin ermittelt, vgl. R. Preussmann, H. Schneider und F. Epple, Arzneimittel-Forschung Band 19, 1059-1073 (1969). Ethylenimin wird bei dem genannten Test mit 4-(4-Nitrobenzyl)pyridin aufgrund einer intensiven violetten Färbung erkannt. Sofern dieser Test negativ ausfällt, ist die Reaktion beendet. Das Reaktionsgemisch kann direkt verwendet werden. Es ist jedoch auch möglich, das Additionsprodukt von Ethylenimin an den Aminoallkylvinylether aus dem Reaktionsgemisch zu isolieren, z.B. durch Entfernen des Wassers.

Die erfindungsgemäßen Alkylenimineinheiten enthaltenden Aminoalkylvinylether sind Monomere, die mit Hilfe von Radikale bildenden Initiatoren polymerisiert werden können. Sie werden daher als Monomere zur Herstellung von Polymeren für den Einsatz in der Papierindustrie verwendet. Polymere mit Molmassen M_{w} bis zu 30 000 eignen sich beispielsweise als Fixiermittel für Störstoffe und Feinstoffe bei der Papierherstellung. Polymerisate von Monomeren der Formel I können außerdem als antimikrobielle Beschichtungsmittel, in Detergentien und zur Behandlung von Metalloberflächen verwendet werden.

### Beispiel 1

In einem Vierhalskolben, der mit Rührer, Thermometer und einer Dosiervorrichtung ausgestattet war, wurden 30 g (0,296 mol) Aminopropylvinylether (CH₂=CH-O-CH₂-CH₂-CH₂-NH₂) in 16 g entionisiertem Wasser gelöst und mit 5 g konzentrierter Schwefelsäure (0,051 mol) gemischt. Der pH-Wert betrug 8,5. Die Mischung wurde in einer Stickstoffatmosphäre unter Rühren auf eine Temperatur von 65 °C erwärmt. Sobald diese Temperatur erreicht war, dosierte man innerhalb von einer Stunde 161 ml einer 60 %igen wässrigen Lösung von Ethylenimin (89,4 g, 2,079 mol). Die Mischung wurde anschließend noch 4 Stunden bei einer Temperatur in dem Bereich von 65 bis 70 °C gerührt. Nach dieser Zeit konnte mit Hilfe des obengenannten Preussmann-Tests kein Ethylenimin mehr im Reaktionsgemisch nachgewiesen werden. Nach Abschluss der Umsetzung betrug der pH-Wert des Reaktionsgemisches 11.

### Beispiel 2

In einem Vierhalskolben mit Intensivrührer, Rückflußkühler und Thermometer wurden 78,2 g (0,772 mol) Aminopropylvinylether (CH₂=CH-O-CH₂-CH₂-CH₂-NH₂) in 41,7 g entionisiertem Wasser gelöst und mit 10 % konzentrierter Schwefelsäure langsam auf einen pH-Wert von 11 eingestellt. Die Mischung wurde in einer Stickstoffatmosphäre unter Rühren auf eine Temperatur von 65 °C erwärmt. Sobald diese Temperatur erreicht war, dosierte man innerhalb von einer Stunde 360 ml einer 60 %igen wässrigen Lösung von Ethylenimin (199,7 g, 4,644 mol) zu. Die Mischung wurde dann noch eine Stunde bei 65 °C gehalten, und anschließend noch vier Stunden bei 85 °C gerührt. Abschließend wurde die Mischung noch drei Stunden unter Rückfluss erhitzt. Nach dieser Zeit konnte mit Hilfe des obengenannten Preussmann-Tests kein Ethylenimin mehr im Reaktionsgemisch nachgewiesen werden.

### Beispiel 3

In einem Vierhalskolben mit Intensivrührer, Rückflußkühler und Thermometer wurden 180 g (1,776 mol) Aminopropylvinylether (CH₂=CH-O-CH₂-CH₂-CH₂-NH₂) in 25 g entionisiertem Wasser gelöst und mit 10 % konzentrierter Schwefelsäure langsam auf einen pH-Wert von 11 eingestellt. Die Mischung wurde in einer Stickstoffatmosphäre unter Rühren auf eine Temperatur von 65 °C erwärmt. Sobald diese Temperatur erreicht war, dosierte man innerhalb von einer Stunde 276 ml einer 60 %igen wässrigen Lösung von Ethylenimin (153,3 g, 3,565 mol) zu. Die Mischung wurde anschließend für 30 Minuten bei 70 °C gerührt. Abschließend wurde die Mischung noch fünf Stunden unter Rückfluss erhitzt. Nach dieser Zeit konnte mit Hilfe des obengenannten Preussmann-Tests kein Ethylenimin mehr im Reaktionsgemisch nachgewiesen werden.

## Patentansprüche

1. Alkylenimineinheiten enthaltende Aminoalkylvinylether der Formel
H₂C = CH - O - X - NH - [Al-]ₙ - H (I),
worin
[Al-]ₙ für n Ethylenimineinheiten steht,
n eine Zahl von 2 bis 20 bedeutet und
X für eine C₃- bis C₄-Alkylengruppe steht,
sowie Salze der Monomeren I mit Mineralsäuren oder organischen Säuren und Quaternierungsprodukte der Monomeren I mit Alkylhalogeniden oder Dialkylsulfaten.

2. Alkylenimineinheiten enthaltende Aminoalkylvinylether nach Anspruch 1, **dadurch gekennzeichnet, dass** in der Formel I
[Al-]ₙ für n Ethylenimineinheiten steht,
n eine Zahl von 3 bis 15 bedeutet und
X für die Gruppe - CH₂ - CH₂ - CH₂ - steht.

3. Verfahren zur Herstellung von Alkylenimineinheiten enthaltenden Aminoalkylvinylethern nach Anspruch 1, **dadurch gekennzeichnet, dass** man an Amino-C₂- bis C₈-alkylvihylether mindestens ein Alkylenimin addiert, wobei man pro Mol des Vinylethers mindestens 1 Mol eines Alkylenimins einsetzt, und die Additionsprodukte zur Herstellung der Salze mit einer Mineralsäure oder einer organischen Säure neutralisiert und sie zur Herstellung der Quatemierungsprodukte mit C₁- bis C₁₈-Alkylhalogeniden oder Dialkylsulfaten umsetzt.

4. Verfahren nach Anspruch 3, **dadurch gekennzeichnet, dass** man an Amino-C₃- bis C₄-alkylvinylether Ethylenimin addiert, wobei man pro Mol des Vinylethers 2 bis 20 Mol Ethylenimin anlagert.

5. Verfahren nach Anspruch 3, **dadurch gekennzeichnet, dass** man an Aminopropylvinylether mit Ethylenimin im Molverhältnis 1 : 3 bis 15 umsetzt.

6. Verwendung der Alkylenimineinheiten enthaltenden Aminoalkylvinylether nach den Ansprüchen 1 und 2 als Monomere zur Herstellung von Polymeren für den Einsatz in der Papierindustrie, als antimikrobielle Beschichtungsmittel, in Detergentien und zur Behandlung von Metalloberflächen.

## Claims

1. An aminoalkyl vinyl ether comprising alkylenimine units and of the formula
H₂C = CH - O - X - NH - [Al-]ₙ - H (I),
in which
[Al-]ₙ is n ethylenimine units,
n is a number from 2 to 20 and
X is a C₃- to C₄-alkylene group,
and salts of the monomers I with mineral acids or organic acids and quaternization products of the monomers I with alkyl halides or dialkyl sulfates.

2. The aminoalkyl vinyl ether comprising alkylenimine units according to claim 1, wherein, in the formula I,
[Al-]ₙ is n ethylenimine units
n is a number from 3 to 15 and
X is the group -CH₂-CH₂-CH₂-.

3. A process for the preparation of an aminoalkyl vinyl ether comprising alkylenimine units according to claim 1, wherein at least one alkylenimine is subjected to an addition reaction with an amino-C₂- to C₆-alkyl vinyl ether, at least 1 mol of an alkylenimine being used per mole of the vinyl ether, and the adducts are neutralized with a mineral acid or an organic acid for the preparation of the salts and they are reacted with C₁- to C₁₈-alkyl halides or dialkyl sulfates for the preparation of the quaternization products.

4. The process according to claim 3, wherein ethylenimine is subjected to an addition reaction with an amino-C₃- to C₄-alkyl vinyl ether, from 2 to 20 mol of ethylenimine being subjected to the addition reaction per mole of the vinyl ether.

5. The process according to claim 3, wherein the aminopropyl vinyl ether is reacted with ethylenimine in the molar ratio of from 1 : 3 to 15.

6. The use of the aminoalkyl vinyl ethers comprising alkylenimine units and according to claims 1 and 2 as monomers for the preparation of polymers for use in the paper industry, as antimicrobial coating materials, in detergents and for the treatment of metal surfaces.

## Revendications

1. Éthers aminoalkylvinyliques contenant des unités alkylène-imine, de formule
H₂C = CH - 0 - X - NH - [Al-]ₙ - H (I),
dans laquelle
[Al-]ₙ représente n unités éthylène-imine,
n représente un nombre valant de 2 à 20 et
X représente un groupe alkylène en C₃-C₄,
ainsi que sels des monomères I avec des acides minéraux ou des acides organiques et produits de quaternisation des monomères I avec des halogénures d'alkyle ou des sulfates de dialkyle.

2. Éthers aminoalkylvinyliques contenant des unités alkylène-imine selon la revendication 1, **caractérisés en ce que** dans la formule I
[Al-]ₙ représente n unités éthylène-imine,
n représente un nombre valant de 3 à 15 et
X représente le groupe -CH₂-CH₂-CH₂-.

3. Procédé pour la préparation d'éthers aminoalkylvinyliques contenant des unités alkylène-imine selon la revendication 1, **caractérisé en ce qu'**on fixe par addition au moins une alkylène-imine sur des éthers aminoalkyl(C₂-C₆)vinyliques, en utilisant au moins 1 mole d'une alkylène-imine par mole de l'éther vinylique, et en neutralisant les produits d'addition, pour la préparation des sels, à l'aide d'un acide minéral ou d'un acide organique, et en les faisant réagir, pour la préparation des produits de quaternisation, avec des halogénures d'alkyle en C₁-C₁₈ ou des sulfates de dialkyle.

4. Procédé selon la revendication 3, **caractérisé en ce qu'**on fixe par addition de l'éthylène-imine sur des éthers amino-alkyl(C₃-C₄)vinyliques, en fixant par addition 2 à 20 moles d'éthylène-imine par mole de l'éther vinylique.

5. Procédé selon la revendication 3, **caractérisé en ce qu'**on fait réagir de l'éther aminopropylvinylique avec de l'éthylène-imine en un rapport molaire de 1:3 à 15.

6. Utilisation des éthers aminoalkylvinyliques contenant des unités alkylène-imine selon les revendications 1 et 2, en tant que monomères pour la préparation de polymères pour l'utilisation dans l'industrie du papier, en tant que produits de revêtement antimicrobiens, dans des détergents et pour le traitement de surfaces métalliques.
